(19) Europäisches Patentamt
European Patent Office
Office européen des brevets

(11) Veröffentlichungsnummer: **0 269 874**
**B1**

(12) # EUROPÄISCHE PATENTSCHRIFT

(45) Veröffentlichungstag der Patentschrift: **11.04.90**

(51) Int. Cl.⁴: **C07C 275/18**, A01N 47/34

(21) Anmeldenummer: **87115955.4**

(22) Anmeldetag: **30.10.87**

(54) **N-Benzoyl-N'-[3,5-dichlor-2-fluor-4-(3-trufluormethyl-phenoxy)-phenyl]-harnstoffe.**

(30) Priorität: **03.11.86 DE 3637403**
**12.12.86 DE 3642466**

(43) Veröffentlichungstag der Anmeldung:
**08.06.88 Patentblatt 88/23**

(45) Bekanntmachung des Hinweises auf die Patenterteilung:
**11.04.90 Patentblatt 90/15**

(84) Benannte Vertragsstaaten:
**BE CH DE ES FR GB GR IT LI NL**

(56) Entgegenhaltungen:
**EP-A- 0 101 990**
**DE-A- 2 537 413**

(73) Patentinhaber: **BASF Aktiengesellschaft,**
**Carl-Bosch-Strasse 38, D-6700 Ludwigshafen(DE)**

(72) Erfinder: **Hofmeister, Peter, Dr.,**
**Bernhard-Humblot-Strasse 12, D-6730 Neustadt(DE)**
Erfinder: **Wild, Jochen, Dr., An der Marlach 7,**
**D-6705 Deidesheim(DE)**
Erfinder: **Liese-Sauer, Thomas, Dr.,**
**Sigmund-Hirsch-Platz 10, D-6940 Weinheim(DE)**
Erfinder: **Neubauer, Hans-Juergen, Dr., B 4,2,**
**D-6800 Mannheim 1(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 269 874 B1

## Beschreibung

Die Erfindung betrifft neue N-Benzoyl-N'-[3,5-dichlor-2-fluor-4-(3-trifluormethyl-phenoxy)-phenyl]-harnstoffe der allgemeinen Formel I

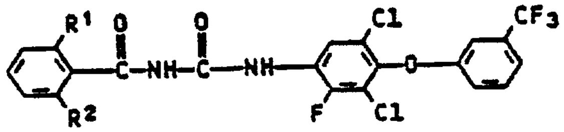

(I)

in der die Substituenten folgende Bedeutung haben:
$R^1$ Fluor, Chlor oder Brom und
$R^2$ Fluor, Chlor oder Wasserstoff.

Außerdem betrifft die Erfindung die Herstellung der Verbindungen I, Schädlingsbekämpfungsmittel, die die Verbindungen I als Wirkstoffe enthalten, sowie eine Verfahren zur Bekämpfung von Schädlingen.

Aus der DE-A-30 44 055 sind N-(Di)halogenbenzoyl-N'-tetrahalogenphenylharnstoffe, aus der DE-A-21 23 236 N-Dihalogenbenzoyl-N'-4-halogen-phenyl-harnstoffe und aus der EP-A-101 990 N-(Di)halogenbenzoyl-N'-[3,5-di(brom)chlor-4-(3-trifluormethyl-phenoxy)-phenyl]-harnstoffe bekannt, die sich zur Bekämpfung von tierischen Schädlingen im Pflanzenschutz eignen. Die Wirkung dieser Verbindungen läßt jedoch zu wünschen übrig.

Der Erfindung lag daher die Aufgabe zugrunde, wirksamere neue N-BenzoylN'-[4-(3-trifluormethyl-phenoxy)-phenyl]-harnstoffe bereitzustellen.

Demgemäß wurden die eingangs definierten neuen N-Benzoyl-N'-[3,5-dichlor-2-fluor-4-(3-trifluormethyl-phenoxy)-phenyl]-harnstoffe I, Verfahren zu deren Herstellung, Schädlingsbekämpfungsmittel, die die Verbindungen I als Wirkstoffe enthalten, sowie ein Verfahren zur Bekämpfung von Schädlingen mit N-Benzoyl-N'-[3,5-dichlor-2-fluor-4-(3-trifluormethyl-phenoxy)-phenyl]-harnstoffen I gefunden.

$R^1$ bedeutet vorzugsweise Fluor oder Chlor.

Unter der Verbindungen I sind diejenigen bevorzugt, in denen $R^1$, $R^2$ = Fluor oder $R^1$, $R^2$ = Chlor bedeuten. Außerdem sind Verbindungen I bevorzugt, in denen $R^1$ = Fluor und $R^2$ = Chlor ist. Ferner sind solche Verbindung I bevorzugt, in denen $R^2$ für Wasserstoff und $R^1$ für Fluor oder Chlor stehen.

Die Verbindungen I sind nach folgenden Methoden erhältlich:

a) durch Umsetzung eines Benzoylisocyanates der allgemeinen Formel II

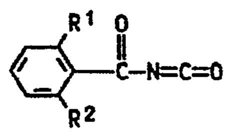

(II)

mit 3,5-Dichlor-2-fluor-4-(3-trifluormethyl-phenoxy)-anilin III

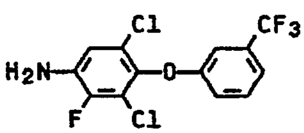

(III)

oder

b) durch Umsetzung eines Benzamides der allgemeinen Formel IV

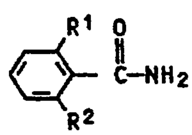

(IV)

mit [3,5-Dichlor-2-fluor-4-(3-trifluormethyl-phenoxy)-phenyl]-iso-cyanat V

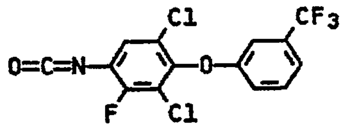

(V).

Die Umsetzung a) verläuft bei Temperaturen von 0 bis 120°C. vorzugsweise 20 bis 80°C und bevorzugt bei 20 bis 60°C, sowie bei Drücken zwischen 1 und 10 bar, vorzugsweise bei Normaldruck. Da die Reaktion unter Wärmeentwicklung (exotherm) verläuft, kann es von Vorteil sein, eine Kühlmöglichkeit vorzusehen.

Zweckmäßigerweise wird das 3,5-Dichlor-2-fluor-4-(3-trifluormethyl-phenoxy)-anilin III in einem Lösungs- oder Verdünnungsmittel vorgelegt unter anschließender Zugabe des Benzoylisocyanats der Formel II. Die Reaktion ist im allgemeinen nach 2 Stunden beendet.

Die Benzoylisocyanate II sind bekannt oder bekannten Methoden herstellbar (J. Org. Chem. 28 (1963), 1805-1811; J. Agr. Food Chem. 21 (1973), 348-354). Das 3,5-Dichlor-2-fluor-(3-trifluormethyl-phenoxy)-anilin III ist neu, jedoch nach bekannten Methoden herstellbar (Organikum, 9. Auflage, 1969, S. 446, 567, VEB Deutscher Verlag der Wissenschaften).

Die Umsetzung b) wird gegebenenfalls in Gegenwart eines Katalysators, bei Temperaturen von 0 bis 140°C, vorzugsweise zwischen 60 und 100°C, sowie bei Drücken zwischen 1 und 10 bar, vorzugsweise bei Normaldruck, durchgeführt. Die Reaktionsdauer liegt im allgemeinen zwischen 2 und 6 Stunden.

Als Katalysatoren eignen sich beispielsweise organische Basen, wie Triethylamin, Pyridin, 4-N,N-Dimethylaminopyridin oder Alkylmetallverbindungen, wie Dibutylzinndiacetat.

Die Benzamide IV sind bekannt oder nach bekannten Methoden (z.B. Houben-Weyl, Methoden der organischen Chemie, Bd. E 5.2, S. 934 ff, Georg-Thieme Verlag, 1985) erhältlich. Das [3,5-Dichlor-2-flour-4-(3-trifluormethyl)-phenoxy]-phenylisocyanat V ist neu, kann jedoch nach bekannten Methoden (z.B. Houben-Weyl, Methoden der organischen Chemie, Bd. E 4, S. 738 ff, Georg Thieme Verlag, 1983) erhalten werden.

Üblicherweise setzt man die Ausgangsstoffe II und III bzw. IV und V in stöchiometrischem Verhältnis ein. Ein Überschüß der einen oder anderen Komponente kann in Einzelfällen aber durchaus vorteilhaft sein.

Die neuen Diphenylether III und V können durch die allgemeine Formel VI

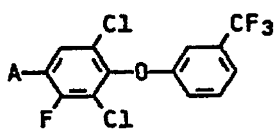

(VI)

(A = Amino, Isocyanato) beschrieben werden und mit üblichen Methoden aus den entsprechenden Nitrobenzolen durch Reduktion hergestellt werden (Houben-Weyl, Methoden der organischen Chemie, Bd. XI/1. S. 360ff (1975)). Die entsprechenden Nitrobenzole ihrerseits werden erhalten durch Umsetzung von 3,5-Dichlor-2,4-difluornitrobenzol mit 3-Trifluormethylphenol bzw. dessen Salze nach bekannten Verfahren. Verbindung III kann in die Verbindung V überführt werden (vgl. Weygard-Hilgetag, Organisch-Chemische Experimentierkunst, 1970; DE-A 25 38 178).

Die Umsetzungen a) und b) werden zweckmäßigerweise in einem Lösungs- oder Verdünnungsmittel ausgeführt. Hierzu sind beispielsweise geeignet; aliphatische und aromatische, gegebenenfalls chlorierte Kohlenwasserstoffe, wie Petrolether, Benzol, Toluol, Xylol, Benzin, Dichlormethan, Chloroform, Tetrachlormethan, 1,2-Dichlormethan und Chlorbenzol, Ether oder Ester wie Diethyl- und Di-n-Butylether, Methyl-tert. -butylether, Tetrahydrofuran, Dioxan und Essigsäureethylester; Ketone, beispielsweise Aceton, Methylethylketon und Methylisopropylketon; ferner Nitrile, wie Acetonitril und Propionitril; aprotische dipolare Lösungsmittel, wie Dimethylformamid, Dimethylsulfoxid oder Pyridin. Auch Gemische dieser Stoffe können als Lösungs- oder Verdünnungsmittel verwendet werden.

Die neuen Verbindung der Formel I fallen teilweise in Form von Ölen an, die sich durch längeres Erwärmen unter vermindertem Druck auf mäßig erhöhte Temperatur ("Andestillieren") von den letzten flüchtigen Anteilen befreien und auf diese Weise reinigen lassen. Erhält man die Verbindungen der Formel I in kristalliner Form, so kann ihre Reinigung durch Umkristallisation erfolgen.

Die N-Benzoyl-N'-[3,5-dichlor-2-fluor-4-(3-trifluormethyl-phenoxy)-phenyl]-harnstoffe der Formel I sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Plutella maculipennis (Kohlschabe), Leucoptera coffeella (Kaffeemotte), Hyponomeuta malinellus (Apfelbaumgespinstmotte), Argyresthia conjugella (Apfelmotte), Sitotroga cerealella (Getreidemotte), Phthorimaea operculella (Kartoffelmotte), Capua reticulana (Apfelschalenwickler), Sparganothis pilleriana (Springwurm), Cacoecia murinana (Tannentriebwickler), Tortrix viridana (Eichenwickler), Clysia ambiguella (Heu- und Sauerwurm), Evetria buoliana (Kieferntriebwickler), Polychrosis botrana (Bekreuzter Traubenwickler), Cydia pomonella (Obstmade), Laspeyresia molesta (Pfirsichtriebbohrer), Laspeyresia funebrana (Pflaumenwickler), Ostrinia nubilalis (Maiszünsler), Loxostege sticticalis (Rübenzünsler), Ephestia kuehniella (Mehlmotte), Chilo suppressalis (Reisstengelbohrer), Galleria mellonella (Wachsmotte), Malacosoma neustria (Ringelspinner), Dendrolimus pini (Kiefernspinner), Thaumatopoea prtiyocampa (Pinienprozessionsspinner), Phalera bucephala (Mondfleck), Cheimatobia brumata (Kleiner Frostspanner), Hibernia defoliaria (Großer Frostspanner), Bupalus piniarius (Kiefernspanner), Hyphantria cunea (Weißer Bärenspinner), Agrotis segetum (Wintersaateule), Agrotis ypsilon (Ypsiloneule), Barathra brassicae (Kohleule), Cirphis unipuncta (Heerwurm), Prodenia litura (Baumwollraupe), Laphygma exigua (Rüben-Heerwurm), Panolis flammea (Forleule), Earias insulana (Baumwollkapselwurm), Plusia gamma (Gammaeule), Alabama argillacea (Baumwollblattwurm), Lymantria dispar (Schwammspinner), Lymantria monacha (Nonne), Pieris brassicae (Kohlweißling), Aporia crataegi (Baumweißling);
aus der Ordnung der Käfer (Coleoptera) beispielsweise Blitophaga undata (Schwarzer Rübenaaskäfer), Melanotus communis (Drahtwurm), Limonius californicus (Drahtwurm), Agriotes lineatus (Saatschnellkäfer), Agriotes obscurus (Humusschnellkäfer), Agrilus sinuatus (Birnbaum-Prachtkäfer), Meligethes aeneus (Rapsglanzkäfer), Atomaria linearis (Moosknopfkäfer), Epilachna varivestis (Mexikanischer Bohnenkäfer), Phyllopertha horticola (Junikäfer), Popillia japonica (Japankäfer), Melolontha melolontha (Feldmaikäfer), Melolontha hippocastani (Waldmaikäfer), Amphimallus solstitialis (Brachkäfer), Crioceris asparagai (Spargelhähnchen), Lema melanopus (Getreidehähnchen), Leptinotarsa decemlineata (Kartoffelkäfer), Phaedon cochleariae (Meerrettich-Blattkäfer), Phyllotreta nemorum (Kohlerdfloh), Chaetocnema tibialis (Rübenflohkäfer), Phylloides chrysocephala (Rape-Flohkäfer), Diabrotica 12-puncta (Südlicher Maiswurzelwurm), Cassida nebulosa (Nebliger Schildkäfer), Bruchus lentis (Linsenkäfer), Brunchus rufimanus (Pferdebohnenkäfer), Brunchus pisorum (Erbsenkäfer), Sitona lineatus (Liniierter Blattrandkäfer), Ortiorrhynchus sulcatus (Gefurchter Lappenrüßler), Otiorrhynchus ovatus (Erdbeerwurzelrüßler), Hylobies abietis (Großer Brauner Rüsselkäfer), Byctiscus betulae (Rebenstecher), Anthonomus pomorum (Apfelblütenstecher), Anthonomus grandis (Kapselkäfer), Ceuthorrhynchus assimilis (Kohlenschotenrüßler), Ceuthorrynchus napi (Großer Kohltriebrüßler), Sitophilus granaria (Kornkäfter), Anisandrus dispar (Ungleicher Holzborkenkäfer), Ips typographus (Buckdrucker), Blastophagus piniperda (Gefurchter Waldgärtner);
aus der Ordnung der Zweiflügler (Diptera) beispielsweise Lycoria pectoralis, Mayetiola destructor (Hessenfliege), Basineura brassicae (Kohlschoten-Gallmücke), Contarinia tritici (Gelbe Weizen-Gallmücke), Haplodiplosis equestris (Sattelmücke), Tipula paludosa (Wiesenschnake), Tipula oleracea (Kohlschnake), Dacus cucurbitae (Melonenfliege), Dacus oleae (Olivenfliege), Ceratitis capitata (Mittelmeerfruchtfliege), Rhagoletis cerasi (Kirschfruchtfliege), Rhagoletis pomonella (Apfelmade), Anastrepha ludens (Mexikanische Fruchtfliege), Oscinella frit (Fritfliege), Phorbia coarctata (Brachfliege), Phorbita antiqua (Zwiebelfliege), Phorbia brassicae (Kleine Kohlfliege), Pegomya hysocyami (Rübenfliege), Anopheles maculipennis, Culex pipiens, Aedes aegypti (Gelbfiebermücke), Aedes vexans, Tabanus bovinus (Rinderbremse), Tipula paludosa (Wiesenschnake), Musca domestica (Stubenfliege), Fannia canicularis (Kleine Stubenfliege), Muscina stabulans, Glossina morsitans (Tsetse-Fliege), Oestrus ovis, Chrysomya macellaria, Chrysomya hominivorax, Lucilia cuprina, Lucilia sericata, Hypoderma lineata;
aus der Ordnung der Hautflügler (Hymenoptera) beispielsweise Athalia rosae (Rübenblattwespe), Hoplocampa minuta (Pflaumensägewespe), Monomorium pharaonis (Pharaoameise), Solenopsis geminata (Feuerameise), Atta sexdens (Blattschneiderameise);
aus der Ordnung der Wanzen (Heteroptera) beispielsweise Nezara viridula (Grüne Reiswanze), Eurygaster integriceps (Asiatische Getreidewande), Blissus leucopterus (Chinch bug), Dysdercus cingulatus (Kapok-Wanze), Dysdercus intermedius (Baumwollwanze), Piesma quadrata (Rübenwanze), Lygus pratensis (Gemeine Wiesenwanze);
aus der Ordnung der Pflanzensauger (Homoptera) beispielsweise Perkinsiella saccharicida (Zuckerrohrzikade), Nilaparvata lugens (Braune Zikade), Empoasca fabae (Kartoffelzikade), Psylla mali (Apfelblattsauger), Psylla piri (Birnblattsauger), Trialeurodes vaporariorum (Weiße Fliege), Aphis fabae (Schwarze Bohnenlaus), Aphis pomi (Grüne Apfellaus), Aphis sambuci (Holunderblattlaus), Aphidula nasturtii (Kreuzdornblattlaus), Cerosipha gossypii (Gurkenblattlaus), Sappaphis mali (Rosige Apfellaus), Sappaphis mala (Mehlige Birnblattlaus), Dysphis radicola (Mehlige Apfelfaltenlaus), Brachycaudus cardui (Große Pflaumenblattlaus), Brevicoryne brassicae (Kohlblattlaus), Phorodon humuli (Hopfenblattlaus), Rhopalomyzus ascalonicus (Zwiebellaus), Myzodes persicae (Grüne Pfirsichlaus), Myzus cerasi (Schwarze Sauerkrischenlause), Dysaulacorthum pseudosolani (Gefleckte Kartoffellaus), Acyrthosiphon onobrychis (Grüne Erbsenlaus), Macrosiphon rosae (Große Rosenblattlaus), Megoura viciae (Wickenlaus), Schizoneura lanuginosa (Birnenblattlaus), Pemphigus bursarius (Salatwurzellaus), Dreyfusia nordmannianae (Tannentrieblaus), Dreyfusia piceae (Weißtannenstammlaus), Adelges laricis (Rote Fichtengallenlaus), Viteus vitifolii (Reblaus);
aus der Ordnung der Termiten (Isoptera) beispielsweise Reticulitermes lucifugus, Calotermes flavicollis, Leucotermes flavipes, Termes natalensis;

aus der Ordnung der Geradflüger (Orthoptera) beispielsweise Forficula auricularia (Gemeiner Ohrwurm), Acheta domestica (Heimchen), Gryllotalpa gryllotalpa (Maulwurfsgrille), Tachycines asynamorus (Gewächshausschrecke), Locusta migratoria (Wanderheuschrecke), Stauronotus maroccanus (Marokkanische Wanderheuschrecke), Schistocerca peregrina (Wanderheuschrecke), Nomadacris septemfasciata (Wanderheuschrecke), Melanoplus spretus (Felsengebirgsheuschrecke), Melanoplus femur-rubrum (Rotbeinige Heuschrecke), Blatta orientalis (Küchenschabe), Blattella germanica (Deutsche Schabe), Periplaneta americana (Amerikanische Schabe), Balbera gigantes (Riesenschabe).

Zur Klasse der Arachnoidea gehören Spinntentiere (Acarina) beispielsweise Ixodes ricinus (Holzbock), Ornithodorus moubata, Amblyomma americanum, Dermacentor silvarum, Boophilus microplus. Tetranychus telarius, Tetranychus pacificus, Paratetranychus pilosus, Bryobia praetiosa.

Zur Klasse der Nematoden zählen beispielsweise Wurzelgallennematoden, z.B. Meloidogyne incognita, Meloidogyne hapla, Meloidogyne javanica, Zysten bildende Nematoden, z.B. Globodera rostochiensis, Heterodera schatii, Heterodera avenae, Hetrodera glycinae, Heterodera triflolii, Stock- und Blattälchen, z.B. Ditylenchus dipsaci, Ditylenchus destructor, Pratylenchus neglectus, Pratylenchus penetrans, Pratylenchus goodeyi, Pratylenchus curvitatus sowie Tylenchorhynchus dubius, Tylenchorhynchus claytoni, Rotylenchus robustus, Heliocotylenchus multicinctus, Radopholus similis, Belonolaimus longicaudatus, Longidorus elongatus, Trichodorus primitivus.

Die Wirkstoffe können als solche, in Form ihrer Formulierungen oder den daraus bereiteten Anwedungsmöglichkeiten, z.B. in Form direkt versprühbaren Lösungen, Pulvern, Suspensionen oder Dispersionen, Emulsionen, Öldispersionen, Pasten, Stäubemitteln, Streumitteln, Granulaten durch Versprühen Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt wie Kerosin oder Dieselöl, ferner Kohlenteeröle sowie Öle pflanzelichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können als Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Disperigier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktiven Stoffe kommen Alkali-, Erdalkali- Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkali, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyde, Kondensationsprodukte des Naphthalins bzw. der Naphthalinsulfonsäure mit Phenol und Formaldehyd, Polyoxyethylenoctylphenolether, ethoxyliertes Isooctylphenol, Octylphenol, Nonylphenon, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einen festen Trägerstoff hergestellt werden. Beispiele für Formulierungen sind:

I. 5 Gew.-Teile der Verbindung Nr. 1 werden mit 95 Gew.-Teilen feinteiligem Kaolin innig vermischt. Man erhält auf diese Weise ein Stäubemittel, das 3 Gew.-% des Wirkstoffs enthält.

II. 30 Gew.-Teile der Verbindung Nr. 5 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

III. 10 Gew.-Teile der Verbindung Nr. 1 werden in einer Mischung gelöst, die aus 90 Gew.-Teilen Xylol, 6 Gew.-Teilen des Anlagerungsproduktes von 8 bis 10 Mol Ethyloenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht.

IV. 20 Gew.-Teile der Verbindung Nr. 5 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht.

V. 80 Gew.-Teile der Verbindung Nr. 1 werden mit 3 Gew.-Teilen des Natriumsalzes der Diisobutylnaphthalin-alpha-sulfonsäure, 10 Gew.-Teilen des Natriumsalzes einer Lignisulfonsäure aus einer Sulfit-Ablauge und 7 Gew.-Teilen pulverförmigen Kieselsäuregel gut vermischt und in einer Hammermühle vermahlen.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Lößß, Ton, Dolomit, Diatomeenerde, Calcium- und Magnesiumsulfat, Magnesiumoxid, gemahlene Kunstoffe, Düngemittel, wie z.B. Ammoniumsulfat, Ammoniumphosphat, Ammoniumnitrat, Harnstoffe, und pflanzliche Produkte, wie Getreidemehe, Baumrinden-, Holz- und Nußschalenmehl, Cellulosepulver und andere feste Trägerstoffe.

Die Formulierungen enthalten im allgemeinen zwischen 0,1 und 95 Gew.-% Wirkstoff, vorzugsweise zwischen 0,5 und 90 Gew.-%.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %. Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Die Aufwandmenge an Wirkstoff beträgt unter Freilandbedingunge 0,2 bis 10, vorzugsweise 0,5 bis 2 kg/ha.

Zu den Wirkstoffen können Öle verschiedenen Typs, Herbizide, Fungizide, andere Schädlingsbekämpfungsmittel, Bakterizide, gegebenenfalls auch erst unmittelbar von der Anwendung (Tankmix), zugesetzt werden. Diese Mittel können zu den erfindungsgemäßen Mitteln im Gewichtsverhältnis 1 : 10 bis 10:1 zugemischt werden.

Beispielsweise können folgende Mittel zugemischt werden: 1,2-Dibrom-3-chlorpropan, 1,3-Dichlorpropen, 1,3-Dichlorpropen + 1,2-Dichlorpropan, 1,2-Dibromethan, 2-sec.-Butyl-phenyl-N-methylcarbamat, o-Chlorphenyl-N-methylcarbamat, 3-Isopropyl-5-methylphenyl-N-methylcarbamat, o-Isopropoxyphenyl-N-methylcarbamat, 3,5-Dimethyl-4-methylmercapto-phenyl-N-methylcarbamat, 4-Dimethylamino-3,5-xylyl-N-methylcarbamat, 2-(1,3-Dioxolan-2-yl)-phenyl-N-methylcarbamat, 1-Naphthyl-N-methylcarbamat, 2,3-Dihydro-2,2-dimethyl-benzofuran-7-yl-N-methylcarbamat, 2,2-Dimethyl-1,3-benzodioxol-4-yl-N-methylcarbamat, 2-Dimethylamino-5,6-dimethyl-4-pyrimidinyl-dimethylcarbamat, 2-Methyl-2-(methylthio)-propionaldehyd-O-(methylcarbamoyl)-oxim, S-Methyl-N[(methylcarbamoyl)-oxyl]-thio-acetimidat, Methyl-N',N'-dimethyl-N-[(methylcarbamoyl)oxy]-1-thiooxamidat, N-(2-Methylchlorphenyl)-N',N'-dimethylformamidin, Tetrachlorthiophen, 1-(2,6-Difluor-benzoyl)-3-(4-chlorphenyl)-harnstoff, O,O-Dimethyl-O-(p-nitrophenyl)-phosphorthioat, O,O-Diethyl-O-(p-nitrophenyl)-phosphorthioat, O-Ethyl-O(p-nitrophenyl)-phenyl-phosphonothioat, O,O-Dimethyl-O-(3-methyl-4-nitrophenyl)-phosphorthioat, O,O-Diethyl-O-(2,4-dichlorphenyl)phosphorthioat, O-Ethyl-O-(2,4-dichlorphenyl)-phenyl-phosphonothioat, O,O-Dimethyl-O-(2,4,5-trichlorphenyl)-phosphorthioat, O-Ethyl-O-(2,4,5-trichlorphenyl)-ethyl-phosphorthioat, O,O-Dimethyl-O-(4-brom-2,5-dichlorphenyl)-phosphorthioat, O,O-Dimethyl-O-(2,5-dichlor-4-jodphenyl)-phosphorthioat, O,O-Dimethyl-O-(3-methyl-4-methylthiophenyl)-phosphorthioat, O-Ethyl-O-(3-methyl-4-methylthiophenyl)-isopropyl-phosphoramidat, O,O-Diethyl-O-(p-methylsulfinyl-phenyl)-phosphorthioat, O-Ethyl-S-phenyl-ethyl-phosphonodithioat, O,O-Diethyl-[2-chlor-1-(2,4-dichlorphenyl)-vinyl]-phosphat, O,O-Dimethyl-[2-chlor-1-(2,4,5-trichlorphenyl)]-vinylphosphat, O,O-Dimethyl-S-(1'-phenyl)-ethylacetat-phosphordithioat, Bis-(dimethylamino)-fluorphosphinoxid, Octamethyl-pyrophosphoramid, O,O,O,O-Tetraethyldithio-pyrophosphat, S-Chlormethyl-O,O-diethylphosphordithioat, O-Ethyl-S,S-dipropyl-phosphordithioat, O,O-Dimethyl-O-2,2-dichlorvinyl-phosphat, O,O-Dimethyl-1,2-dibrom-2,2-dichlorethylphosphat, O,O-Dimethyl-2,2,2-trichlor-1-hydroxy-ethylphosphonat, O,O-Dimethyl-S-[1,2-biscarbethoxy-ethyl-(1)]-phosphordithioat, O,O-Dimethyl-O-(1-methyl-2-carbmethoxy-vinyl)-phosphat, O,O-Dimethyl-S-(N-methyl-carbamoyl-methyl)-phosphordithioat, O,O-Dimethyl-S-(N-methylcarbamoyl methyl)-phosphorthioat, O,O-Dimethyl-S-(N-methoxyethyl-carbamoylmethyl)-phosphordithioat, O,O-Dimethyl-S-(N-formyl-N-methyl-carbamoyl-methyl)-phosphordithioat, O,O-Dimethyl-O-[1-methyl-2-(methyl-carbamoyl)-vinyl]-phosphat, O,O-Dimethyl-O-[(1-methyl-2-dimethylcarbamoyl)-vinyl]-phosphat, O,O-Dimethyl-O-[(1-methyl-2-chlor-2-diethylcarbamoyl)-vinyl]-phosphat, O,O-Diethyl-S-(ethylthiomethyl)-phosphordithioat, O,O-Diethyl-S-[(p-chlorphenylthio)-methyl]-phosphordithioat, O,O-Dimethyl-S-(2-ethylthioethyl)-phosphorthioat, O,O-Dimethyl-S-(2-ethylthioethyl)-phosphordithioat, O,O-Dimethyl-S-(2-ethylsulfinyl-ethyl)-phosphorthioat, O,O-Diethyl-S-(2-ethylthioethyl)-phosphordithioat, O,O-Diethyl-S-(2-ethylsulfinyl-ethyl)-phosphorthioat, O,O-Diethyl-thiophosphoryliminophenyl)-acetonitril, O,O-Diethyl-S-(2-chlor-1-phthalimidoethyl)-phosphordithioat, O,O-Diethyl-S-[6-chlor-benzoxazolon-(2)-yl(3)]-methyldithiophosphat, O,O-Dimethyl-S-[2-methoxy-1,3,4-thiadiazol-5-[4H]-onyl-(4)-methyl]-phosphordithioat, O,O-Diethyl-O-[3,5,6-trichlor-pyridyl-(2)]-phosphorthioat, O,O-Diethyl-O-(2-pyrazinyl)-phosphorthioat, O,O-Diethyl-O-[2-isopropyl-4-methyl-pyrimidinyl(6)]-phosphorthioat, O,O-Diethyl-O-[2-(diethylamino)-6-methyl-4-pyrimidinyl]-thionophosphat, O,O-Dimethyl-S-(4-oxo-1,2,3-benzotriazin-3-[4H]-yl-methyl)-phosphordithioat, O,O-Dimethyl-S-[(4,6-diamino-1,3,5-triazin-2-yl)-methyl]-phosphordithioat, O,O-Diethyl-(1-phenyl-1,2,4-triazol-3-yl)-thionophosphat, O,S-Dimethyl-phosphor-amidothioat, O,S-Dimethyl-N-acetyl-phosphoramidothioat, alpha-Hexachlorcyclohexan, 1,1-Di-(p-methoxyphenyl)-2,2,2-trichlor-ethan, 6,7,8,9,10,10-Hexachloro-1,5,5a,6,9,9a-hexahydro-6,9-methano-2,4,3-benzodioxa-thiepin-3-oxid, Pyrethrine, DL-2-Allyl-3-methyl-cyclopenten-(2)-on-(1)-yl-(4)-DL-cis,-trans-chrysanthemat, 5-Benzyl-furyl-(3)-methyl-DL-cis,-trans-chrysanthemat, 3-Phenoxybenzyl(±)-cis,-trans-2,2-dimethyl-3-(2,2-dichlorvinyl)-cylcopropancarboxylat, alpha-Cyano-3-phenoxybenzyl(±)-cis,-trans-2,2-dimethyl-3-(2,2-dichlorvinyl)-cyclopropancarboxylat, (s)-alpha-Cyano-3-phenoxybenzyl-cis(1R,3R)-2,2-dimethyl-3-(2,2-dibromvinyl)-cyclopropancarboxylat, 3,4,5,6-Tetrahydrophthalimidoethyl-DL-cis,-trans-chrysanthemat, 2-Methyl-5-(2-propinyl)-3-furylmethyl-chrysanthemat, (alpha-Cyano-3-phenoxybenzyl)-alpha-isopropyl-4-chlorphenylacetat.

Herstellungsbeispiel

N-2,6-Difluorbenzoyl-N'-[3,5-dichlor-2-fluor-4-(3-trifluormethylphenoxy)-phenyl]-harnstoff (Beispiel 1)

23,9 g (0,11 mol) 3,5-Dichlor-2,4-difluornitrobenzol werden mit 17,3 g (0,10 mol) 3-Trifluormethylphenol und 4,8 g (0,12 mol) NaOH in 150 ml wasserfreiem Dimethylsulfoxid 10 Stunden bei 120°C gerührt. Nach dem Abkühlen gießt man in Eiswasser und extrahiert mit Petrolether. Man erhält 34 g Rohprodukt, welches mittels Säulenchromatographie an Kieselgel mit Toluol/Essigester (8:2) gereinigt wird; Ausbeute 28,3 g 3,5-Dichlor-2-fluor-4-(3-trifluormethyl-phenoxy)-nitrobenzol.

28,3 g des zuvor erhaltenen Produktes werden in 150 ml wasserfreiem Tetrahydrofuran mit 2 g Raney-Nickel bei Raumtemperatur und 0,3 bar $H_2$-Druck hydriert. Man trennt vom Katalysator ab, engt ein und erhält so 26,4 g Rohprodukt von 3,5-Dichlor-2-fluor-4-(3-trifluormethyl-phenoxy)-anilin, welches mittels Säulenchromatographie an Kieselgel mit Toluol/ Essigester (9:1) als Laufmittel gereinigt wird.

Zu einer Lösung von 4,5 g (0,013 mol) des zuvor erhaltenen Produktes in 50 ml wasserfreiem Tetrahydrofuran tropft man bei Raumtemperatur 2,4 g (0,013 mol) 2,6-Difluorbenzoylisocyanat. Anschließend wird 2 Stunden bei 45°C gerührt und nach Abkühlen auf 0°C mit 100 ml n-Pentan versetzt und abgesaugt. Nach Trocknung erhält man 4,55 g (67 %) N-2,6-Difluorbenzoyl-N'-[3,5-dichlor-2-fluor-4-(3-trifluormethyl-phenoxy)-phenyl]-harnstoff vom Schmelzpunkt 186 bis 188°C (Verbindung 1 der folgenden Tabelle).

Tabelle

| Verbindung Nr. | $R^1$ | $R^2$ | Fp (°C) |
|---|---|---|---|
| 1 | F | F | 186–188 |
| 2 | F | H | |
| 3 | F | Cl | |
| 4 | Cl | Cl | |
| 5 | Cl | H | 189 |
| 6 | Br | F | |
| 7 | Br | Cl | |
| 8 | Br | H | |

Anwendungsbeispiele

In den folgenden Beispielen wurden die erfindungsgemäßen Verbindungen bzw. sie enthaltende Mittel hinsichtlich ihrer Wirkung auf Schädlinge mit den nachstehenden Verbindungen des Standes der Technik verglichen.

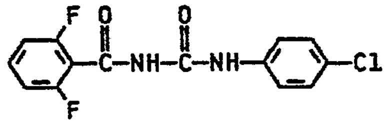

bekannt aus
DE-A-21 23 236
Anspruch 69

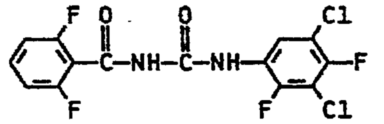

bekannt aus
DE-A-30 44 055
als Verbindung B.2
auf Seite 9

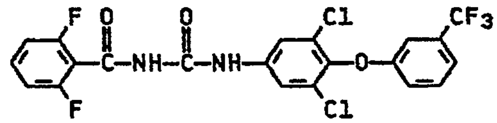

bekannt aus
EP-A-101 990 als
Verbindung Nr. 20

Die Konzentrationen, bei denen die untersuchten Verbindungen eine 100 %ige Mortalität bzw. Hemmung bewirken, sind die jeweiligen Minimalkonzentrationen. Bei jeder Konzentration wurden mindestens 2 Versuche angesetzt.

Beispiel A

Musca domestica (Stubenfliegen), Chemosterilisation

Ca. 100 schlüpffähige Puppen von Stubenfliegen werden in Folienbehälter mit 8,5 1 Volumen gesetzt. Sie erhalten hier 2 g Futter, bestehend aus
6 Teilen Kristallzucker
6 Teilen Trockenmilch
1 Teil Eipulver,
dem 20 mg des Wirkstoffs in organischer Lösung zugefügt werden (= 1 Gew.%). Das Lösungsmittel wird anschließend entfernt. Wasser wird in ausreichender Menge auf Zellstoff in 100 ml Bechern geboten. Die Tiere bleiben ca. 8 Tage in diesen Käfigen bei 22°C und Tag- und Nachtrhythmus. Am 8. Tag gibt man ein Eiablagegefäß mit in Magermilch getränktem Zellstoff in die Käfige. Hier erfolgt innerhalb der nächsten 24 Stunden die Eiablage. Beim Einstellen der Eiablagegefäße wird die Fraßgiftwirkung anhand der toten Fliegen beurteilt. Nach 3 bis 4 Tagen wird die Entwicklung der Eier auf der Milchwatte beurteilt.

| | | | |
|---|---|---|---|
| Beispiel 1 | | 10 ppm | 100% Mortalität |
| | | 4 ppm | ca. 80% Mortalität |
| Vergleichsmittel | A | 4000 ppm | 100% Mortalität |
| | B | 400 ppm | ca. 80% Mortalität |
| | C | 100 ppm | ca. 90% Mortalität |

Beispiel B

Tineola bisselliella (Kleidermotte), Zuchtversuch

Die Versuche werden auf 3,2 g eines Wollstoffes (10 x 20 cm) durchgeführt. Man behandelt zu Versuchsbeginn den Wollstoff mit der acetonischen Wirkstofflösung, dabei müssen 3,2 ml aufgetropft werden. Anschließend stäubt man etwas Kleidermotten-Zuchtfutter auf den Wollstoff und fügt 30 mg Motteneier hinzu. Der Stoff wird nun zusammengerollt und in einem geschlossenen 1-l-Glas bei 22 bis 24°C aufbewahrt. Die Fraßaktivität der Larven kann nach ca. 8 Wochen festgestellt werden. Nach weiteren 14 Tagen beginnen die Falter zu schlüpfen.

| | | | |
|---|---|---|---|
| Beispiel 1 | | 0,2 ppm | 100% Mortalität |
| Vergleichsmittel | A | 0,4 ppm | 100% Mortalität |
| | B | 0,2 ppm | 100% Mortalität |
| | C | 1 ppm | 100% Mortalität |

Beispiel C

Blatta orientalis (Schabe), Zuchtversuch

Die Prüfung erfolgt in 1 l-Gläsern an 50 Schaben im ersten Larvenstadium. Ein Gestell aus 5 horizontalen Pappscheiben (6 x 6 cm) bietet den Tieren Schlupfmöglichkeiten. Der Wirkstoff wird über das Futter aufgenommen. Dieses besteht aus 20 g kalt angerührtem Karottenbrei in Petrischalen (Ø 5 cm). Zusätzlich erhalten sie Wasser auf Zellstoff in Plastikbechern (Inhalt 25 ml). Die Beobachtungszeit erstreckt sich über zwei Häutungsphasen (3 Wochen). Dabei muß wöchentlich mit frisch zubereitetem Brei nachgefüttert werden. Die Beurteilung erfolg wöchentlich.

| | | | |
|---|---|---|---|
| Beispiel 1 | | 4 ppm | 98% Mortalität |
| Vergleichsmittel | A | 10 ppm | 88% Mortalität |
| | B | 40 ppm | 100% Mortalität |
| | C | 100 ppm | 0% Mortalität |

Beispiel D

Tetranychus telarius (Spinnmilbe) Versuche mit gezählten Weibchen

Getropfte Buschbohnen werden in der Spritzkabine auf dem Drehteller mit der wäßrigen Wirkstoffaufbereitung tropfnaß gespritzt. Nach dem Abrocknen stanzt man mit einem Korkbohrer Blattstücke von 25 mm Durchmesser aus den Spreiten. Sie werden auf Zellstoffstücke gelegt, welche an den Seiten in Wasser hängen. Die Blattstanzstücke werden mit je 10 adulten Weibchen belegt. Nach 5 und 10 Tagen erfolgt die Auswertung, wobei Eier, Larven und Adulte ausgezählt werden.

| | | | |
|---|---|---|---|
| Beispiel 1 | | 0,02% | ca. 95% Mortalität |
| | | 0,01% | ca. 85% Mortalität |
| Vergleichsmittel | A | 0,4% | 0% Mortalität |
| | B | 0,1% | 0% Mortalität |

Beispiel E

Agrotis ypsilon (Erdraupe), Entwicklungshemmung (Prüfung auf behandeltem Nährboden)

100 g des Standard-Nährbodens für Agrotis werden in 250 ml-Becher gefüllt und warm mit der wäßrigen Wirkstoffaufbereitung sorgfältig gemischt. Nach Erkalten belegt man jedes Gefäß mit 10 Raupen des dritten Larvenstadiums (1,5 bis 1,8 cm) und lagert sie bei 23°C. Beurteilt wird Verpuppung und Schlupf der Falter.

| | | | |
|---|---|---|---|
| Beispiel 1 | | 0,04 ppm | 100% Mortalität |
| | | 0,02 ppm | ca. 90% Mortalität |
| Vergleichsmittel | A | 10 ppm | ca. 95% Mortalität |
| | B | 1 ppm | ca. 80% Mortalität |
| | C | 0,1 ppm | 100% Mortalität |

Beispiel F

Aedes aegypti (Gelbfiebermücke); Zuchtversuch

200 ml Leitungswasser in 250 ml Plastikbecher werden mit der Wirkstoffaufbereitung versetzt und darauf mit 20 bis 30 Moskitolarven im dritten bis vierten Larvenstadium besetzt. Die Gefäße stehen bei 25°C. Beobachtet werden Verpuppung und Schlüpfen der Imagines, welches nach 10 bis 12 Tagen erfolgt. Während der Beobachtungszeit wird einmal mit gepulvertem Zierfischfutter (Tetramin®) gefüttert.

| | | | |
|---|---|---|---|
| Beispiel 1 | | 0,002 ppm | 100% Mortalität |
| Vergleichsmittel | A | 0,004 ppm | 100% Mortalität |
| | B | 0,004 ppm | 100% Mortalität |
| | C | 0,004 ppm | 100% Mortalität |

**Patentansprüche für die Vertragsstaaten: BE, CH, DE, FR, GB, IT, LI, NL, GR**

1. N-Benzoyl-N'-[3,5-dichlor-2-fluor-4-(3-trifluormethyl-phenoxy)-phenyl]-harnstoffe der Formel I

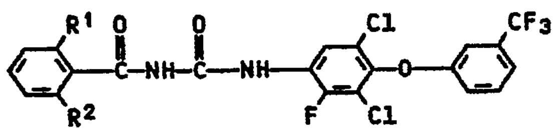

(I)

in der die Substituenten folgende Bedeutung haben:
$R^1$ Fluor, Chlor oder From und
$R^2$ Fluor, Chlor oder Wasserstoff.

2. N-Benzoyl-N'-[3,5-dichlor-2-fluor-4-(3-trifluormethyl-phenoxy)-phenyl]-harnstoffe der Formel I nach Anspruch 1, in der die Substituenten $R^1$ und $R^2$ Fluor bedeuten.

3. N-Benzoyl-N'-[3,5-dichlor-2-fluor-4-(3-trifluormethyl-phenoxy)-phenyl]-harnstoffe der Formel I nach Anspruch 1, in der die Substituenten $R^1$ und $R^2$ Chlor bedeuten.

4. N-Benzoyl-N'-[3,5-dichlor-2-fluor-4-(3-trifluormethyl-phenoxy)-phenyl]-harnstoffe der Formel I nach Anspruch 1, in der die Substituenten $R^1$ Fluor und $R^2$ Chlor bedeuten.

5. N-Benzoyl-N'-[3,5-dichlor-2-fluor-4-(3-trifluormethyl-phenoxy)-phenyl]-harnstoffe der Formel I nach Anspruch 1, in der die Substituenten $R^1$ Chlor und $R^2$ Wasserstoff bedeuten.

6. N-Benzoyl-N'-[3,5-dichlor-2-fluor-4-(3-trifluormethyl-phenoxy)-phenyl]-harnstoffe der Formel I nach Anspruch 1, in der die Substituenten $R^1$ Fluor und $R^2$ Wasserstoff bedeuten.

7. Verfahren zur Herstellung von N-Benzoyl-N'-[3,5-dichlor-2-fluor-4-(3-trifluormethyl-phenoxy)-phenyl]-harnstoffen der Formel I nach Anspruch 1, dadurch gekennzeichnet, daß man

a) ein Benzoylisocyanat der Formel II

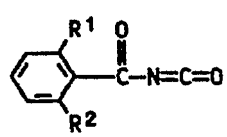

(II)

mit 3,5-Dichlor-2-fluor-4-(3-trifluormethyl-phenoxy)-anilin III

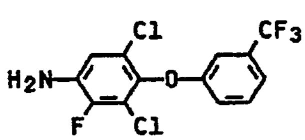

(III)

oder
b) ein Benzamid IV

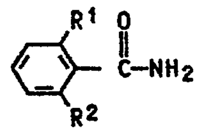

(IV)

mit [3,5-Dichlor-2-fluor-4-(3-trifluormethyl-phenoxy)-phenyl]-isocyanat V

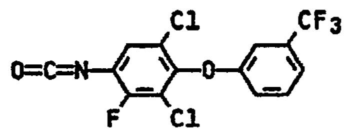

(V)

in an sich bekannter Weise umsetzt.

8. Schädlingsbekämpfungsmittel, enthaltend 0,1 bis 95 Gew.% eines N-Benzoyl-N'-[3,5-dichlor-2-fluor-4-(3-trifluormethyl-phenoxy)-phenyl]-harnstoffes der Formel I gemäß Anspruch 1 neben üblichen Trägern.

9. Verfahren zur Bekämpfung von Schädlingen, dadurch gekennzeichnet, daß man die Schädlinge und/oder die von Schädlingen freizuhaltenden Flächen und/oder Räume mit einer für Schädlinge wirksamen Menge eines N-Benzoyl-N'-[3,5-dichlor-2-fluor-4-(3-trifluormethyl-phenoxy)-phenyl]-harnstoffes der Formel I gemäß Anspruch 1 behandelt.

10. Diphenylether der allgemeinen Formel VI

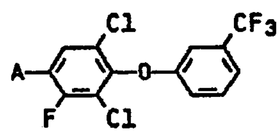

(VI)

in der der Substituent A Amino oder Isocyanato bedeutet.

**Patentansprüche für den Vertragsstaat: ES**

1. Verfahren zur Herstellung von N-Benzoyl-N'[3,5-dichlor-2-fluor-4-(3-trifluormethyl-phenoxy)-phenyl]-harnstoffen der Formel I

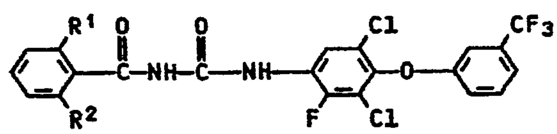

(I)

in der die Substituenten folgende Bedeutung haben:
$R^1$ Fluor, Chlor oder Brom und
$R^2$ Fluor, Chlor oder Wasserstoff,
dadurch gekennzeichnet, daß man

a) ein Benzoylisocyanat der Formel II

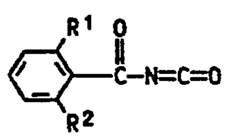

(II)

mit 3,5-Dichlor-2-fluor-4-(3-trifluormethyl-phenoxy)-anilin III

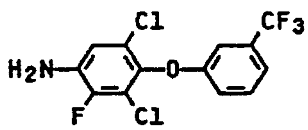

(III)

bei Temperaturen von 0 bis 120°C und Drücken von 1 bis 10 bar oder

b) ein Benzamid IV

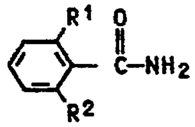

(IV)

mit [3,5-Dichlor-2-fluor-4-(3-trifluormethyl-phenoxy)-phenyl]-isocyanat V

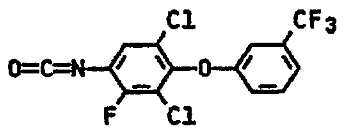

(V)

gegebenenfalls in Gegenwart eines Katalysators bei Temperaturen von 0 bis 140°C und Drücken von 1 bis 10 bar umsetzt.

2. Verfahren zur Herstellung von N-Benzoyl-N'-[3,5-dichlor-2-fluor-4-(3-trifluormethyl-phenoxy)-phenyl]-harnstoffen der Formel I nach Anspruch 1, in der die Substituenten $R^1$ und $R^2$ Fluor bedeuten.

3. Verfahren zur Herstellung von N-Benzoyl-N'-[3,5-dichlor-2-fluor-4-(3-trifluormethyl-phenoxy)-phenyl]-harnstoffen der Formel I nach Anspruch 1, in der die Substituenten $R^1$ und $R^2$ Chlor bedeuten.

4. Verfahren zur Herstellung von N-Benzoyl-N'-[3,5-dichlor-2-fluor-4-(3-trifluormethyl-phenoxy)-phenyl]-harnstoffen der Formel I nach Anspruch 1, in der die Substituenten $R^1$ Fluor und $R^2$ Chlor bedeuten.

5. Verfahren zur Herstellung von N-Benzoyl-N'-[3,5-dichlor-2-fluor-4-(3-trifluormethyl-phenoxy)-phenyl]-harnstoffen der Formel I nach Anspruch 1, in der die Substituenten $R^1$ Chlor und $R^2$ Wasserstoff bedeuten.

6. Verfahren zur Herstellung von N-Benzoyl-N'-[3,5-dichlor-2-fluor-4-(3-trifluormethyl-phenoxy)-phenyl]-harnstoffen der Formel I nach Anspruch 1, in der die Substituenten $R^1$ Fluor und $R^2$ Wasserstoff bedeuten.

7. Schädlingsbekämpfungsmittel, enthaltend 0,1 bis 95 Gew.% eines N-Benzoyl-N'-[3,5-dichlor-2-fluor-4-[3-trifluormethyl-phenoxy)-phenyl]-harnstoffes der Formel I gemäß Anspruch 1 neben üblichen Trägern.

8. Verfahren zur Bekämpfung von Schädlingen, dadurch gekennzeichnet, daß man die Schädlinge und/oder die von Schädlingen freizuhaltenden Flächen und/oder Räume mit einer für Schädlinge wirksamen Menge eines N-Benzoyl-N'-[3,5-dichlor-2-fluor-4-(3-trifluormethyl-phenoxy)-phenyl]-harnstoffes der Formel I gemäß Anspruch 1 behandelt.

9. Verfahren zur Herstellung von Diphenylethern der allgemeinen Formel VI

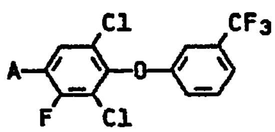

(VI)

in der der Substituent A Amino oder Isocyanato bedeutet, dadurch gekennzeichnet, daß man das entsprechende Nitroderivat von VI (A = $NO_2$) in an sich bekannter Weise umsetzt.

**Claims for the Contracting States: BE, CH, DE, FR, GB, GR, IT, LI, NL**

1. An N-benzoyl-N'-[3,5-dichloro-2-fluoro-4-(3-trifluoromethylphenoxy)-phenyl]-urea of the formula I

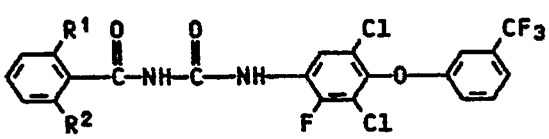

(I)

where $R^1$ is fluorine, chlorine or bromine and $R^2$ is fluorine, chlorine or hydrogen.

2. An N-benzoyl-N'-[3,5-dichloro-2-fluoro-4-(3-trifluoromethylphenoxy)-phenyl]-urea of the formula I as claimed in claim 1, where $R^1$ and $R^2$ are both fluorine.

3. An N-benzoyl-N'-[3,5-dichloro-2-fluoro-4-(3-trifluoromethylphenoxy)-phenyl]-urea of the formula I as claimed in claim 1, where $R^1$ and $R^2$ are both chlorine.

4. An N-benzoyl-N'-[3,5-dichloro-2-fluoro-4-(3-trifluoromethylphenoxy)-phenyl]-urea of the formula I as claimed in claim 1, where $R^1$ is fluorine and $R^2$ is chlorine.

5. An N-benzoyl-N'-[3,5-dichloro-2-fluoro-4-(3-trifluoromethylphenoxy)-phenyl]-urea of the formula I as claimed in claim 1, where $R^1$ is chlorine and $R^2$ is hydrogen.

6. An N-benzoyl-N'-[3,5-dichloro-2-fluoro-4-(3-trifluoromethylphenoxy)-phenyl]-urea of the formula I as claimed in claim 1, where $R^1$ is fluorine and $R^2$ is hydrogen.

7. A process for the preparation of an N-benzoyl-N'-[3,5-dichloro-2-fluoro-4-(3-trifluoromethylphenoxy)phenyl]-urea of the formula I as claimed in claim 1, by

a) reacting a benzoyl isocyanate of the formula II

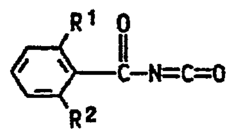

(II)

with 3,5-dichloro-2-fluoro-4-(3-trifluoromethylphenoxy)-aniline III

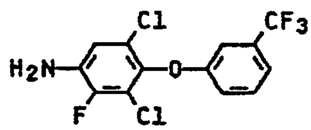

(III)

or

b) reacting a benzamide IV

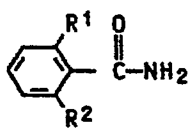

(IV)

with 3,5-dichloro-2-fluoro-4-(3-trifluoromethylphenoxy)-phenyl isocyanate V

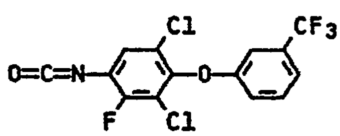

(V)

in a known manner.

8. A pesticide containing from 0.1 to 95% by weight of an N-benzoyl-N'-[3,5-dichloro-2-fluoro-4-(3-trifluoromethylphenoxy)-phenyl]-urea of the formula I as claimed in claim 1, in addition to conventional carriers.

9. A process for combating pests, wherein the pests and/or the areas and/or spaces to be kept free from pests are treated with an effective amount for pests of an N-benzoyl-N'-[3,5-dichloro-2-fluoro-4-(3-trifluoromethyl- phenoxy)-phenyl]-urea of the formula I as claimed in claim 1.

10. A diphenyl ether of the general formula VI

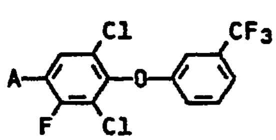

(VI)

where A is amino or isocyanato.

**Claims for the Contracting State: ES**

1. A process for the preparation of an N-benzoyl-N'-[3,5-dichloro-2-fluoro-4-(3-trifluoromethylphenoxy)-phenyl]-urea of the formula I

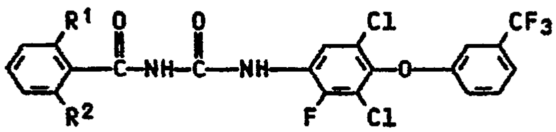

(I)

where $R^1$ is fluorine, chlorine or bromine and $R^2$ is fluorine chlorine or hydrogen, wherein

a) a benzoyl isocyanate of the formula II

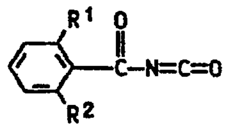

(II)

is reacted with 3,5-dichloro-2-fluoro-4-(3-trifluoromethylphenoxy)-aniline III

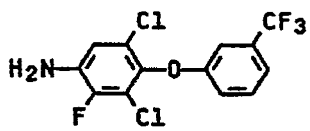

(III)

at from 0 to 120°C and under from 1 to 10 bar or

b) a benzamide IV

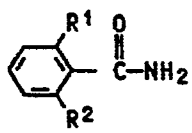

(IV)

is reacted with 3,5-dichloro-2-fluoro-4-(3-trifluoromethylphenoxy)-phenyl isocyanate V

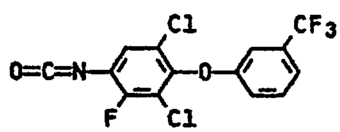

(V)

in the presence or absence of a catalyst at form 0 to 140°C and under form 1 to 10 bar.

2. A process for the preparation of an N-benzoyl-N'-[3,5-dichloro-2-fluoro-4-(3-trifluoromethylphenoxy)-phenyl]-urea of the formula I as claimed in claim 1, where $R^1$ and $R^2$ are both fluorine.

3. A process for the preparation of an N-benzoyl-N'-[3,5-dichloro-2-fluoro-4-(3-trifluoromethylphenoxy)-phenyl]-urea of the formula I as claimed in claim 1, where $R^1$ and $R^2$ are both chlorine.

4. A process for the preparation of an N-benzoyl-N'-[3,5-dichloro-2-fluoro-4-(3-trifluoromethylphenoxy)-phenyl]-urea of the formula I as claimed in claim 1, where $R^1$ and $R^2$ is chlorine.

5. A process for the preparation of an N-benzoyl-N'-[3,5-dichloro-2-fluoro-4-(3-trifluoromethylphenoxy)-phenyl]-urea of the formula I as claimed in claim 1, where $R^1$ and $R^2$ is hydrogen.

6. A process for the preparation of an N-benzoyl-N'-[3,5-dichloro-2-fluoro-4-(3-trifluoromethylphenoxy)-phenyl]-urea of the formula I as claimed in claim 1, where $R^1$ and $R^2$ is hydrogen.

7. A pesticide containing from 0.1 to 95% by weight of an N-benzoyl-N'-[3,5-dichloro-2-fluoro-4-(3-trifluoromethylphenoxy)-phenyl]-urea of the formula I as claimed in claim 1, in addition to conventional carriers.

8. A process for combating pests, wherein the pests and/or the areas and/or spaces to be kept free from pests are treated with an effective amount for pests of an N-benzoyl-N'-[3,5-dichloro-2-fluoro-4-(3-trifluoromethylphenoxy)-phenyl]-urea of the formula I as claimed in claim 1.

9. A process for the preparation of a diphenyl ether of the formula VI

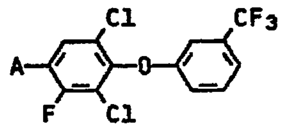

(VI)

where A is amino or isocyanato, wherein the corresponding nitro derivative of VI (A = $NO_2$) is reacted in a known manner.

**Revendications pour les Etats contractants: BE, CH, FR, GB, GR, IT, LI, NL**

1. N-benzoyl-N'-[3,5-dichloro-2-fluoro-4-(3-trifluoro-méthyl-phénoxy)-phényl]-urées de formule I

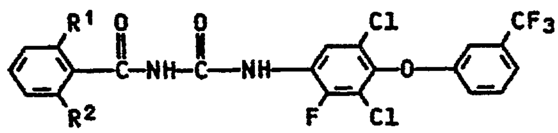

(I)

dans laquelle
$R^1$ représente le fluor, le chlore ou le brome et $R^2$ représente le fluor, le chlore ou l'hydrogène.

2. N-benzoyl-N'-[3,5-dichloro-2-fluoro-4-(3-trifluoro-méthyl-phénoxy)-phényl]-urées de formule I selon la revendication 1 dans laquelle $R^1$ et $R^2$ représentent le fluor.

3. N-benzoyl-N'-[3,5-dichloro-2-fluoro-4-(3-trifluoro-méthyl-phénoxy)-phényl]-urées de formule I selon la revendication 1 dans laquelle $R^1$ et $R^2$ représentent le chlore.

4. N-benzoyl-N'-[3,5-dichloro-2-fluoro-4-(3-trifluoro-méthyl-phénoxy)-phényl]-urées de formule I selon la revendication 1 dans laquelle $R^1$ représente et le fluor et $R^2$ le chlore.

5. N-benzoyl-N'-[3,5-dichloro-2-fluoro-4-(3-trifluoro-méthyl-phénoxy)-phényl]-urées de formule I selon la revendication 1 dans laquelle $R^1$ représente le chlore et $R^2$ l'hydrogène.

6. N-benzoyl-N'-[3,5-dichloro-2-fluoro-4-(3-trifluoro-méthyl-phénoxy)-phényl]-urées de formule I selon la revendication 1 dans laquelle $R^1$ représente le fluor et $R^2$ l'hydrogène.

7. N-benzoyl-N'-[3,5-dichloro-2-fluoro-4-(3-trifluoro-méthyl-phénoxy)-phényl]-urées de formule I selon la revendication 1, caractérisé par le fait que l'on fait réagir de manière connue en soi:

a) un isocyanate de benzoyle de formule II

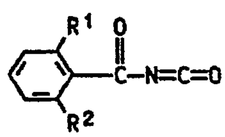

(II)

avec la 3,5-dichloro-2-fluoro-4-(3-trifluorométhyl-phénoxy)-aniline III

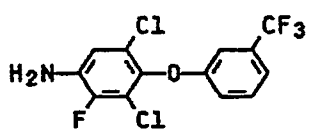

(III)

ou bien
un benzamide IV

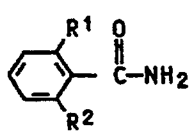

(IV)

avec l'isocyanate de 3,5-dichloro-2-fluoro-4-(3-trifluorométhyl-phénoxy)-phényle V

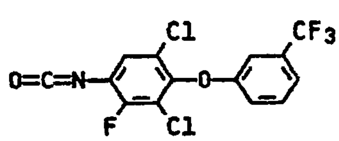

(V)

8. Produits pesticides contenant de 0,1 à 95% en poids d'une N-benzoyl-N'-[3,5-dichloro-2-fluoro-4-(3-trifluoro-méthyl-phénoxy)-phényl]-urées de formule I selon la revendication 1 avec des véhicules usuels.

9. Procédé pour combattre les parasites, caractérisé par le fait que l'on traite les parasites et/ou les surfaces et/ou locaux à protéger contre les parasites par une quantité efficace sur les parasites d'une N-benzoyl-N'-[3,5-dichloro-2-fluoro-4-(3-trifluoro-méthyl-phénoxy)-phényl]-urées de formule I selon la revendication 1.

10. Ethers diphényliques de formule générale VI

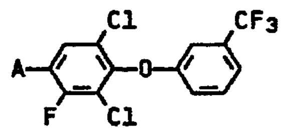

(VI)

dans laquelle le symbole A représente un groupe amino ou isocyanato.

**Revendications pour l'Etat contractant: ES**

1. Procéde de préparation des N-benzoyl-N'-[3,5-dichloro-2-fluoro-4-(3-trifluorométhyl-phénoxy]-urées de formule I

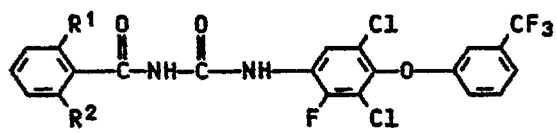

(I)

dans laquelle $R^1$ représente le fluor, le chlore ou le brome, et $R^2$ représente le fluor, le chlore ou l'hydrogène, caractérisé par le fait que:

a) on fait réagir un isocyanate de benzoyle de formule II

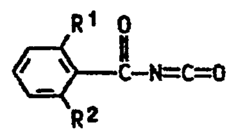

(II)

avec la 3,5-dichloro-2-fluoro-4-(3-trifluorométhyl-phénoxy)-aniline III

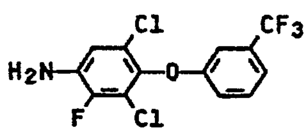

(III)

à des températures de 0 à 120 C et des pressions de 1 à 10 bar, ou bien

b) on fait réagir un benzamide IV

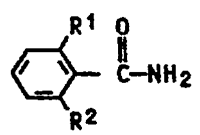

(IV)

avec l'isocyanate de 3,5-dichloro-2-fluoro-4-(3-trifluorométhyl-phénoxy)-phényle V

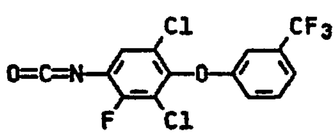

(V)

éventuellement en présence d'un catalyseur, à des températures de 0 à 140 C et des pressions de 1 à 10 bar.

2. Procéde de préparation des N-benzoyl-N'-[3,5-dichloro-2-fluoro-4-(3-trifluorométhyl-phénoxy]-urées de formule I selon la revendication 1 dans laquelle $R^1$ et $R^2$ représentent le fluor.

4. Procéde de préparation des N-benzoyl-N'-[3,5-dichloro-2-fluoro-4-(3-trifluorométhyl-phénoxy]-urées de formule I selon la revendication 1 dans laquelle $R^1$ représente le fluor et $R^2$ le chlore.

5. Procéde de préparation des N-benzoyl-N'-[3,5-dichloro-2-fluoro-4-(3-trifluorométhyl-phénoxy]-urées de formule I selon la revendication 1 dans laquelle $R^1$ représente le chlore et $R^2$ l'hydrogène.

6. Procéde de préparation des N-benzoyl-N'-[3,5-dichloro-2-fluoro-4-(3-trifluorométhyl-phénoxy]-urées de formule I selon la revendication 1 dans laquelle $R^1$ représente le fluor et $R^2$ l'hydrogène.

7. Produits pesticides contenant de 0,1 à 95% en poids d'une N-benzoyl-N'-[3,5-dichloro-2-fluoro-4-(3-trifluorométhyl-phénoxy]-urées de formule I selon la revendication 1 avec des véhiclues usuels.

8. Procédé pour combattre les parasites, caractérisé par le fait que l'on traite les parasdites et/ou les surfaces et/ou locaux à protéger contre les parasites par une quantité efficace sur les parasites d'une N-benzoyl-N'-[3,5-dichloro-2-fluoro-4-(3-trifluorométhyl-phénoxy]-urées de formule I selon la revendication 1.

9. Procédé de préparation des éthers diphényliques de formule générale VI

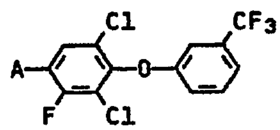

(VI)

dans laquelle le symbole A représente un groupe amino ou isocyanato, caractérisé par le fait que l'on convertit le dérivé nitré correspondant de VI ($A = NO_2$) de manière connue en soi.